# EUROPEAN PATENT SPECIFICATION

(11) **EP 1 425 379 B1**
(45) Date of publication and mention of the grant of the patent: **26.07.2006**
(21) Application number: 02758587.6
(22) Date of filing: 11.09.2002
(51) Int. Cl.: C12M 3/06, A61L 27/14, A61L 27/38

(54) **METHOD AND STRUCTURE FOR GROWING LIVING ORGANIC TISSUE**
VERFAHREN UND ANLAGE ZUR ZÜCHTUNG VON ORGANISCHEN GEWEBEKULTUREN
PROCEDE ET STRUCTURE POUR CULTIVER UN TISSU ORGANIQUE VIVANT

(30) Priority: 11.09.2001 GB 0121986
(43) Date of publication of application: 09.06.2004
(73) Proprietor: ISIS INNOVATION LIMITED, Summertown, Oxford OX2 7SG (GB)
(72) Inventor: TRIFFITT, J. T., University of Oxford, Botnar R.C., Oxford OX3 7LD (GB); XIA, Zhidao, University of Oxford, Botnar R.C., Oxford OX3 7LD (GB); YE, Hua, D.E.S. University of Oxford, Oxford OX1 3PJ (GB); CUI, Zhanfeng, D.E.S. University of Oxford, Oxford OX1 3PJ (GB)
(74) Representative: Smith, Samuel Leonard
(86) International application number: PCT/GB2002/004126
(87) International publication number: WO 2003/022985

(56) References cited:
- EP-A- 1 031 356
- WO-A-96/30492
- US-A- 3 883 393
- US-A- 4 661 458
- US-A- 5 759 830
- US-A1- 2001 039 047
- HUTMACHER D W: "Scaffolds in tissue engineering bone and cartilage" BIOMATERIALS, ELSEVIER SCIENCE PUBLISHERS BV., BARKING, GB, vol. 21, no. 24, 15 December 2000 (2000-12-15), pages 2529-2543, XP004217417 ISSN: 0142-9612
- GERMAIN L ET AL: "TISSUE ENGINEERING OF THE VASCULAR SYSTEM: FROM CAPILLARIES TO LARGER BLOOD VESSELS" MEDICAL AND BIOLOGICAL ENGINEERING AND COMPUTING, PETER PEREGRINUS LTD. STEVENAGE, GB, vol. 38, no. 2, March 2000 (2000-03), pages 232-240, XP000914405 ISSN: 0140-0118
- ARNAOUT W S ET AL: "Development of Bioartificial Liver: Bilirubin Conjugation in Gunn Rats" JOURNAL OF SURGICAL RESEARCH, ACADEMIC PRESS INC., SAN DIEGO, CA, US, vol. 48, no. 4, April 1990 (1990-04), pages 379-382, XP002954972 ISSN: 0022-4804
- MINICHIELLO M M ET AL: "A PERFUSION SYSTEM DEVELOPED FOR 31P NMR STUDY OF MELANOMA CELLS AT TISSUE-LIKE DENSITY" MAGNETIC RESONANCE IN MEDICINE, ACADEMIC PRESS, DULUTH, MN, US, vol. 10, no. 1, 1 April 1989 (1989-04-01), pages 96-107, XP000007545 ISSN: 0740-3194

## Description

The present invention relates to a method and structure for growing living organic tissue, in particular where a scaffold is provided on which cells of the tissue may be seeded and nutrient fluid is provided to grow the tissue.

Engineered tissue involves the seeding of appropriate cells into a scaffold (also known as a matrix) to form a bio-construct. The bio-construct is then cultured with defined conditions for a certain period of time. In particular, it is known to grow small sections of skin tissue in this way.

The tissue is grown in a generally planar form and immersed in nutrient fluid such that nutrients and oxygen may diffuse to all of the cells of the tissue.

Various types of scaffold or matrix are well known and include collagen gels. To function effectively as a scaffold or matrix, it is essential that the scaffold or matrix has the appropriate porosity and surface area for attachment of cells. Where the scaffold is fibrous in nature, it is important that the fibres have a relatively small diameter such that they provide a large surface area, whilst taking up a relatively small volume. The fibres are then arranged with a density/porosity to allow cell attachment and growth between the fibres.

It is also known to provide a scaffold in which the fibres of the scaffold are themselves hollow, thereby allowing diffusion of nutrient fluid along the inside of the fibres between the cells. Although this offers some advantages over scaffolds using solid fibres, transport of fluids is still relatively limited such that tissue is still grown only as a generally laminar sheet layer.

In order to allow growth of tissue having a more three dimensional like structure, documents such as US 4,661,458 and GB 2,178,447A propose arranging the two-dimensional layer structures one on top of the other. However, where layers of scaffold are required in this way, construction is relatively complicated and there are considerable limitations to the overall form and shape of the three-dimensional tissue growth.

EP-A-1 152 053, which is relevant under Article 54(3) EPC, describes a process for producing a three-dimensional bioartificial tissue having viable cells in or on a matrix, and by which cells and matrix can be cultivated into a tissue or a precursor of a tissue, a vascularised tissue of biological materials, obtained by the process, and an experimental reactor for scientific purposes and for producing clinically usable tissues and organs. A vessel is inserted into the artificial biological tissue at the beginning of this production. It can be inserted into the matrix before it is inoculated with the cells desired for the artificial biological tissue. A vessel of natural origin from a human or animal can be used as the vessel. It is also possible to use an artificial vessel, particularly one of a biologically compatible polymer.

ARNAOUT W S ET AL.: "Development of Bioartificial Liver: Bilirubin Conjugation in Gunn Rats" JOURNAL OF SURGICAL RESEARCH, ACADEMIC PRESS INC., SAN DIEGO, CA, US, vol. 48, no. 4, April 1990 (1990-04), pages 379-382, XP002954972 ISSN: 0022-4804 describes a bioartificial liver using micro-carrier-attached hepatocytes and a bioreactor. The bioreactor consists of an intracapillary chamber made up of porous cellulose acetate hollow fibres enclosed in a polycarbonate module forming an extracapillary chamber. This chamber formed around the hollow fibres or capillaries is filled with micro-carrier-attached hepatocytes.

In view of the above, it is an object of the present invention to enable the provision of engineered tissue structures which have other desired shapes and sizes.

According to the present invention, there is provided a method of growing living organic tissue including:
freely defining a volume of desired shape and size;
providing a plurality of membrane capillaries along freely choosable paths interspersed through the volume, each membrane capillary including a respective membrane wall which is permeable to nutrients and oxygen and which defines an internal passageway for conveying a nutrient fluid wherein the membrane capillaries are bio-degradable or bioresorbable;
forming a scaffold in the volume around the plurality of membrane capillaries, the scaffold being suitable for seeding of tissue cells and tissue growth;
seeding cells of the tissue in the scaffold; and
providing a flow of nutrient fluid through the plurality of membrane capillaries.

According to the present invention, there is also provided a structure for growing living organic tissue including:
a scaffold on which cells of the tissue may be seeded and on which the tissue may be grown; and
a plurality of membrane capillaries, separate from the scaffold, each membrane capillary including a respective membrane wall defining an internal passageway for conveying a nutrient fluid, wherein the membrane walls are permeable to nutrients and oxygen and the plurality of membrane capillaries are interspersed through the scaffold along freely chosen paths such that the shape of the scaffold may be freely chosen with the plurality of membrane capillaries interspersed throughout the scaffold so as to enable the tissue to be grown throughout the scaffold wherein the membrane capillaries are bio-degradable or bioresorbable.

In this way, there may furthermore be provided a tissue structure including:
living organic tissue; and
a plurality of artificial membrane capillaries each membrane capillary including a respective membrane wall defining an internal passageway for conveying a nutrient fluid, wherein the membrane walls are permeable to nutrients and oxygen and the plurality of membrane capillaries are interspersed through the tissue along freely chosen paths such that the shape of the tissue may be freely chosen with the plurality of membrane capillaries interspersed throughout the tissue so as to support life of the tissue wherein the membrane capillaries are bio-degradable or bioresorbable.

In this way, the tissue may be grown on a scaffold of any desired shape and size. Whereas the previously known thin sections of skin tissue could be considered as being substantially 2-dimensional, according to the present invention, in contrast, 3-dimensional scaffolds and tissue structures may be provided. By virtue of the membrane capillaries, supply of nutrients to the cells deep inside the tissue construct is no longer limited by diffusion through the scaffold from the outer surface. The blood circulation network, upon which natural tissue depends, is not normally present in engineered tissues. Previously, nutrient supply diffusion to cells inside the tissue was a limiting factor. However, by embedding a membrane capillary network in the scaffold so as to provide nutrients to cells inside the tissue, scaffolds and tissues may be provided with any desired shape and size. Once the tissue has been grown, it may be implanted into an animal or human as required. Depending on the requirements and application of the tissue, the membrane capillaries may be arranged to degrade or be absorbed during their artificial growth or after being implanted.

It will be appreciated that the term tissue is intended to cover all forms of living organic material, such as cartilage, bones, blood vessels, marrow, muscles, nerves and organs.

Preferably, the scaffold is formed with a plurality of fibres or particles of relatively small diameter compared to the membrane capillaries.

In this way, the scaffold provides a large surface area and structure within which the cells may be seeded and grown, whereas the membrane capillaries provide relatively large channels for the nutrients and oxygen. As a result, nutrients and oxygen may be carried deep inside the scaffold so as to allow tissue to be grown which is relatively thick.

In this way, the scaffold and/or tissue may have a relatively large extent in three mutually orthogonal directions.

Of course, the relatively large diameter membrane capillaries maybe accompanied by smaller diameter membrane capillaries approaching the physiological capillary size which are connected to them.

Preferably, the membrane capillaries are not constrained to follow paths within laminar sheet layers. In this respect, reference to laminar sheet layers covers both planar and curved layers.

According to known techniques, the supply of nutrients and oxygen is dependent on diffusion through a relatively thin layer of scaffold or along the relatively small diameter fibres making up the scaffold. It is only possible to build up what might be considered a three-dimensional structure by rolling these layers or providing the layers one on top of the other.

Where, according to the invention, the membrane capillaries are interposed along freely chosen paths and are not constrained to follow paths within laminate sheet layers, the scaffold and tissue may be formed with any desired shape.

Preferably, at least one of the membrane capillaries is provided to follow a path which is neither in nor parallel with the laminar sheet layer in which the path of another of the membrane capillaries lies.

In other words, the membrane capillaries do not have to follow the known layered structure. Where one capillary is in a particular direction which can be considered to lie in a laminar sheet layer, another membrane capillary may follow a path in an unrelated direction, for instance skew with the first capillary. Hence, there is great flexibility in the paths of the capillaries and, hence, the shape of scaffold and tissue which can be serviced by the membrane capillaries.

Preferably at least one of the membrane capillaries is provided along a convoluted path not constrained within a laminate sheet layer.

In other words, each membrane capillary may follow any desired path through the scaffold and tissue and need not follow a layered structure as was the case previously.

Preferably, the plurality of membrane capillaries are provided with omni-directional paths.

Thus, the capillaries may each have a path determined solely by the needs of the tissue which they are to support.

Membrane capillaries of different diameters may be used in the same structure.

Preferably, in the method of growing the living organic tissue, the volume is defined by choosing a desired shape and size for a mould.

Hence, for whatever shape and size of tissue that is required, an appropriate mould is formed.

Preferably, the method includes the step of providing the plurality of membrane capillaries in the mould.

Thus, having determined the shape and size of tissue required and having provided an appropriate mould, the membrane capillaries can be positioned within the mould along paths appropriate to support growth of the tissue throughout its volume.

Preferably, the scaffold material is introduced into the mould around the plurality of membrane capillaries.

The scaffold is thus formed in the desired shape of the mould with the membrane capillaries interspersed through it with the desired spacing and pattern. It is possible to mix cells with the scaffold material before it is introduced into the mould. In this way, the cells are easily and fully interspersed throughout the scaffold before growth is started.

Preferably, at least some of the membrane capillaries are open at opposite ends so as each to provide a passage for flow of nutrient through the tissue such that nutrient fluid provides nutrients to the tissue.

In this way, the membrane capillaries carry nutrient fluid through the tissue. Fresh fluid enters at one end, whereas fluid from which nutrients have diffused to the cells flows out of the other end.

At least some of the membrane capillaries may be blocked at one end such that nutrient fluid may be supplied to the open end so as to provide nutrients to the tissue.

In this way, nutrient fluid does not flow through the membrane capillaries, but is provided into the membrane capillaries, flows within the capillaries into the tissue and then is distributed through the capillary walls into the tissue cells.

At least some of the membrane capillaries may be blocked at both ends.

In this way, fluid inside the capillaries provides a diffusion path of less resistance.

At least some of the plurality of membrane capillaries may have membranes permeable to metabolic waste and be suitable for conveying fluid containing metabolic waste away from the tissue.

In this way, where nutrient fluid flows from one end of a membrane capillary to the opposite end, nutrients may diffuse and disperse from the fluid to surrounding cells of the tissue whereas metabolic waste may be absorbed into the fluid and, hence, be extracted from the membrane capillary and be disposed of.

An additional plurality of membrane capillaries may be provided having membranes permeable to metabolic waste and which are interspersed through the scaffold so as to convey metabolic waste fluid away from the tissue.

Hence, membrane capillaries may be provided to remove metabolic waste in the same way that membrane capillaries are positioned to supply nutrients.

At least some of the additional plurality of membrane capillaries may be blocked one of opposite ends such that metabolic waste fluid may be drawn away from the tissue out of the open ends.

This is particularly applicable where membrane capillaries with blocked ends are provided for supplying nutrients. In particular, one set of membrane capillaries provides nutrients to the cells and another set of membrane capillaries extract metabolic waste from the cells.

The structure for growing the organic tissue may be provided with a waste extractor for drawing metabolic waste fluid from the additional plurality of membrane capillaries.

Similarly, the structure may include a nutrient fluid supply for providing nutrient fluid to the membrane capillaries.

The membrane capillaries may be constructed from any suitable material, for instance such as polylactic acid (PLA), polyglycolic acid (PGA), polylactic-coglycolic acid (PLGA) or polycaprolactone.

Preferably, the membrane capillaries are of porous walls having pores, for instance being constructed from a material having pores which, as the membrane capillaries degrade, increase in size so as to provide an increased supply of nutrient fluid to the tissue.

In this way, as the culture progresses and the cell density inside the tissue increases, the larger pore size will allow a higher transport of nutrients to the tissue and, therefore, help to meet the increased demand for nutrients.

Preferably, endothelial cells may be introduced in the membrane capillaries with desired biochemical signalling to stimulate growth of blood vessels in place of the membrane capillaries.

Normally, but not exclusively, these endothelial cells can be introduced at a later stage.

The endothelial cells attach within the membrane capillaries and themselves grow initially to form a monolayer. Thus, as the membrane capillaries degrade, they are replaced by the growth of blood vessels.

Of course, growth of tissue into the volumes taken up by the membrane capillaries once they have degraded/absorbed will be relatively slow and, hence, the resulting channels will allow flow of nutrient fluid. Thus, it is also possible to implant the tissue structure and allow blood vessels to develop naturally subsequent to implantation.

Preferably, the membrane capillaries are between substantially 10µm and 2mm diameter, more preferably 0.1mm and 1mm.

In this way, the capillaries are of sufficient cross-sectional area to carry a good flow of fluid and yet do not take up an undue amount of volume in the tissue structure. For organ growth diameters up to 5mm may be necessary, but for other tissue growth diameters up to 2mm may be sufficient.

Preferably, the membrane capillaries are spaced no more than substantially 5mm apart.

It will be appreciated that nutrients and metabolic waste have to diffuse between the cells and the membrane capillaries. Hence, if the cells are too far from a membrane capillary, there will be insufficient receipt of nutrients and removal of waste.

Preferably, the membrane capillaries are spaced with adjacent respective walls no closer than 0.5mm apart.

Preferably at least some of the membrane capillaries extend through the scaffold parallel to one another.

Thus, the capillaries may be arranged in an ordered array through the tissue, for instance extending between two opposite sides of the tissue.

Alternatively, at least some of the membrane capillaries may follow respective circuitous paths through the scaffold.

In this way, the membranes may follow shapes of the tissue and start and finish at arbitrary points with respect to the tissue. The circuitous paths may be part of an organised overall form or may be random within the volume of the tissue. Irrespective, in order to provide proper life support for the tissue, no part of the tissue should be away from a membrane capillary by more than a predetermined distance. This could be achieved by arranging the membrane capillaries in a predetermined organised fashion or providing sufficient numbers/lengths of membrane capillaries such that, the minimum distance will be achieved.

The invention will be more clearly understood from the following description, given by way of example only, with reference to the accompanying drawings, in which:
Figure 1 illustrates schematically a mould for a structure according to the present invention;
Figure 2 illustrates a cross-section through the assembled mould of Figure 1;
Figure 3 illustrates a bio-construct according to the present invention produced from the mould of Figure 1;
Figure 4 illustrates the structure of one type of scaffold;
Figure 5 illustrates a membrane capillary for use with the present invention;
Figure 6 illustrates an arrangement of membrane capillaries in a structure according to the present invention;
Figure 7 illustrates an arrangement of membrane capillaries in a structure according to the present invention;
Figure 8 illustrates an arrangement of membrane capillaries in a structure according to the present invention;
Figure 9 illustrates an arrangement of membrane capillaries in a structure according to the present invention;
Figure 10 illustrates an arrangement of membrane capillaries blocked at both ends in a structure according to the present invention;
Figure 11 illustrates interconnected membrane capillaries in a structure according to the present invention;
Figure 12 illustrates an overall system for growing living organic tissue; and
Figure 13 illustrates schematically a bioreactor;
Figure 14 illustrates a bioreactor used for experiments;
Figure 15 illustrates a bioreactor perfusion system;
Figure 16 illustrates alamar blue assay results;
Figure 17 illustrates lactate production results;
Figure 18 illustrates pH value results;
Figures 19(a) and 19(b) illustrate CO₂ and O₂ partial pressure results;
Figures 20(a) to 20(d) illustrate K+, Na+, Ca++ and Cl- concentration results;
Figure 21 illustrates living/dead cell staining results;
Figure 22(a) to 22(d) illustrates H & E staining of harvested cells and tissue; and
Figure 23 illustrates SEM pictures.

It is already known to provide scaffolds in which cells may be seeded so as to form a bio-construct which may be cultured under predefined conditions. Such scaffolds are described for instance in R.C. Thompson et al, Polymer Scaffold Processing; Principle of Tissue Engineering 2nd Edition, Edited by R.L. Lanza, R Langer and J. Vacanti (2000).

With a preferred embodiment of the present invention, a suitable polymer material, preferably bio-degradable or bioresorbable and with a degradation/adsorption rate chosen according to the needs of the user, is provided in a solution which may be poured into a mould. The solution goes on to form a gel forming a porous interconnected network within which tissue may be grown.

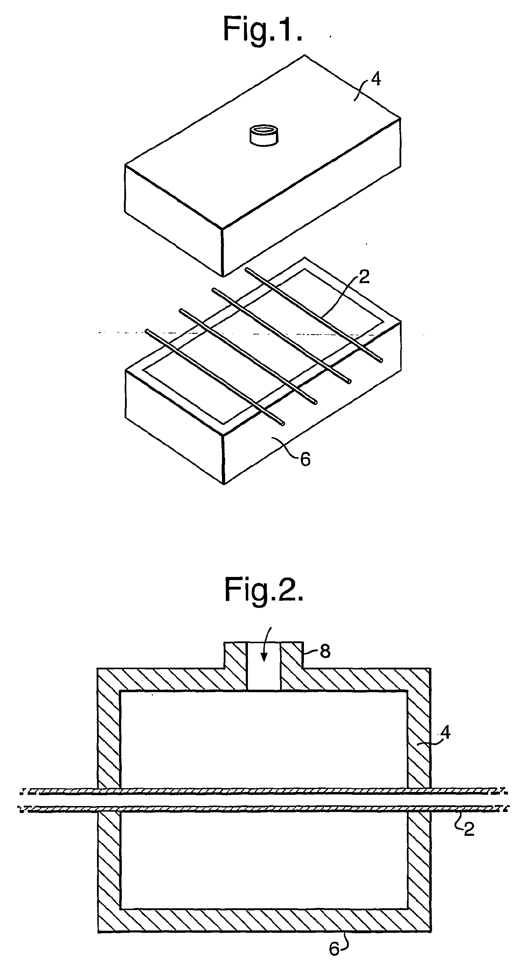

As illustrated schematically in Figure 1, during the moulding stage membrane capillaries 2 may be provided through the mould, in this case provided by the two sections 4 and 6.

Figure 2 is a cross section through the assembled mould of Figure 1 along one of the membrane capillaries 2. As illustrated by the arrow, the material for the scaffold may be provided through the mould opening 8.

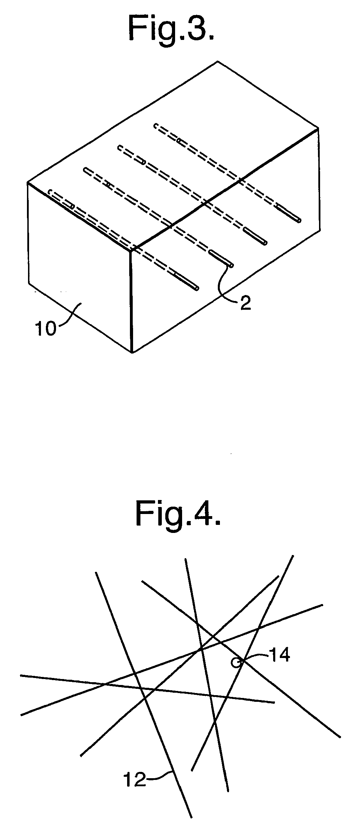

As a result, a scaffold 10 may be provided as illustrated in Figure 3 having a number of membrane capillaries 2 passing through it. The capillaries 2, which will be described further below, are permeable to nutrients and oxygen. Hence, by providing, for instance by pumping, nutrient fluid through the capillaries 2 from one end to the other, nutrients may be provided to the internal volume of the scaffold 10. As illustrated, the scaffold 10 has been removed from the mould 4,6. However, it should be noted that it is not necessary to remove the scaffold from the mould. The mould could itself form part of the "bioreactor" to retain the gel and go on to support tissue growth.

As described above, the scaffold 10 forms a structure made up of a porous interconnected network. This is illustrated schematically in Figure 4. The strands 12 forming the interconnecting structure have relatively small diameters and leave spaces in which cells 14 may be seeded. In particular, the strands 12 provide support for the seeded cells 14 and the cells 14 are then able to divide and grow supported by the structure of the scaffold.

According to previous techniques, supply of nutrients to the cells 14 is reliant on flow of nutrients through the structure or, where hollow scaffold fibres are used, the relatively narrow passageways provided by the scaffold fibres. However, of course, for larger volumes of tissue in which the cells fill the structure, it becomes impossible for sufficient nutrients to find their way to the cells and diffuse the required distances. The membrane capillaries 2 provide channels for fluid into central regions of the scaffold and tissue.

The membrane capillaries themselves are permeable to nutrients such that the nutrients may move from inside the capillary to the cells of the tissue and from one location to another.

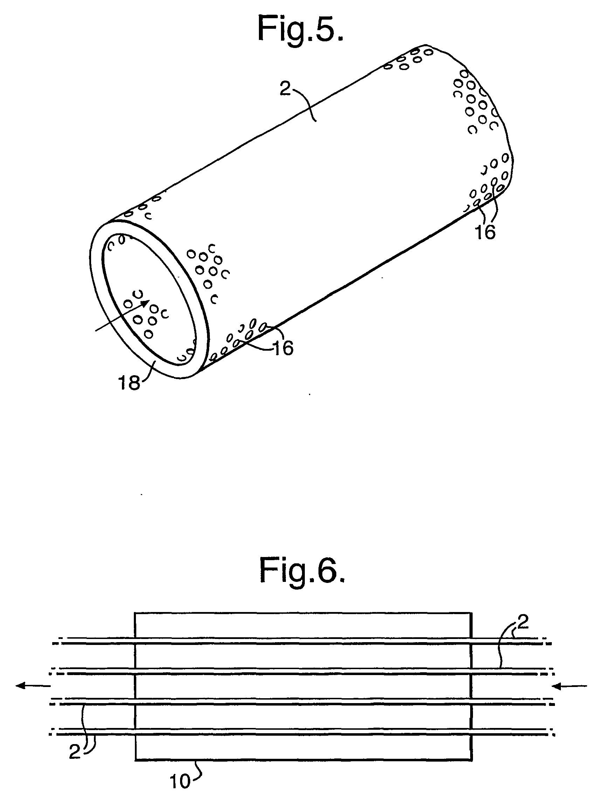

Figure 5 illustrates schematically a capillary 2 which is porous. Nutrient fluid may be provided into the capillary 2 as illustrated by the arrow such that nutrients can diffuse (or even be pumped) through pores 16 of the capillary wall 18. The membrane capillaries 2 may themselves form part of the scaffold for the tissue such that cells of the tissue grow on the outer surface of the capillaries 2. In this respect, of course, nutrients may flow from the pores 16, but the tissue cells cannot grow into the capillary 2 through the pores 16 due to the relative size of the cells and the pores. Preferably, the pores at least start with cross-sections of less than 1 µm, or indeed 0.1 µm.

In some embodiments, it may be preferred that the cells do not seed or grow on the membrane capillaries 2 themselves. This makes it easier to remove the capillaries if necessary.

For laboratory work, where the membrane capillaries may remain embedded in the tissue, it is possible to use non-bio-degradable and bio-compatible materials for the capillaries 2. Suitable materials for the capillaries include poly sulfone and poly ether sulfone. Where the tissue is to be implanted in an animal or human body, of course, it is preferred, if not necessary, for the membrane capillaries 2 to be removed from the tissue. In a preferred embodiment, this is achieved by constructing the membrane capillaries from bio-degradable materials. Examples of such materials include polylactic acid (PLA), polyglycolic acid (PGA) and polycaprolactone. Indeed, PLA hollow fibres are known from Chinese J Reparative and Reconstructive Surgery, 2000 Vol 14(2), page 40, Materials Fabrication of Tissue Engineered Peripheral Nerve in Vitro/WANG Guang-lin, YANG Zhi-ming, XIE Hui-qi et al.

Production of the capillaries having appropriate permeability by virtue of the pore sizes may be achieved using known techniques and adjusting the sizes of the capillaries and the pores according to the requirements of the tissue growth.

The material properties of the capillary membranes 2 can also be adjusted to vary the rate of bio-degradation. In this respect, it will be appreciated that the growth of larger tissue structures will require longer periods of development. In this case, the membrane capillaries will be required to remain intact for longer periods. Similarly, in some applications, a user may require the membrane capillaries 2 to remain intact until after the tissue structure has been implanted into the host animal or human body. Again, in this case, the membrane capillaries 2 will require material properties such that they remain intact longer.

To vary the degradation rate, it is possible to alter the material properties, including composition, of the membrane capillaries 2. Alternatively or additionally, the thickness of the walls 18 of the membrane capillaries 2 can be varied. In particular, membrane capillaries 2 having thicker walls 18, will, of course, remain intact for longer periods of time.

For particular reasons with regard to the tissue structure being grown, it is possible to provide, within the same scaffold and tissue structure, membrane capillaries 2 having different respective rates of degradation. This might have particular application when, as to be described below, natural blood vessels are developed in the tissue.

Where a membrane capillary 2 is bio-degradable, it can be arranged such that, as it degrades, the pores 16 become enlarged. By arranging the membrane capillaries 2 in this way, they progressively provide an increased flow of nutrients to the surrounding tissue. This is highly advantageous since, of course, over time, the tissue will grow and, hence, its requirements for nutrients will increase. It is envisaged that normal tissue remodelling will give optimal tissue nutrition after in-vivo implantation.

It will be appreciated that the basic scaffold, such as scaffold 10 in Figure 3, may be moulded in any shape or size, depending on the application. There are then various ways in which the membrane capillaries 2 may be arranged in that scaffold. These arrangements can be broadly considered in various groups.

As illustrated in Figure 6, each membrane capillary 2 is open at both ends and extends through the tissue and scaffold 10. Oxygenated nutrient fluid flows through each membrane capillary 2 as illustrated. On its path through the tissue and scaffold 10, nutrients and oxygen disperse from the membrane capillary 2 to the surrounding tissue.

At this point, it is worth noting that, of course, the living cells of the tissue produce metabolic waste, including lactate and carbon dioxide. Thus, in the preferred embodiment, this metabolic waste should be removed from the tissue. By arranging the material properties of at least some of the membrane capillaries 2 to be permeable to the metabolic waste, it is possible to use these membrane capillaries 2 to remove the metabolic waste from the tissue. In practice, in most cases, the membrane capillaries 2 will, when permeable for nutrients, be permeable for the metabolic waste.

Thus, referring again to Figure 6, as the fluid flows through the membrane capillaries 2, nutrients and oxygen pass from the fluid to the surrounding cells, whereas metabolic waste is absorbed/dissolved/diffused into the fluid and, hence, removed from the structure.

Alternatively, some of the membrane capillaries 2 can be used for conveying nutrient fluid, whereas others of the membrane capillaries 2 can convey a neutral fluid intended for picking up and removing the metabolic waste.

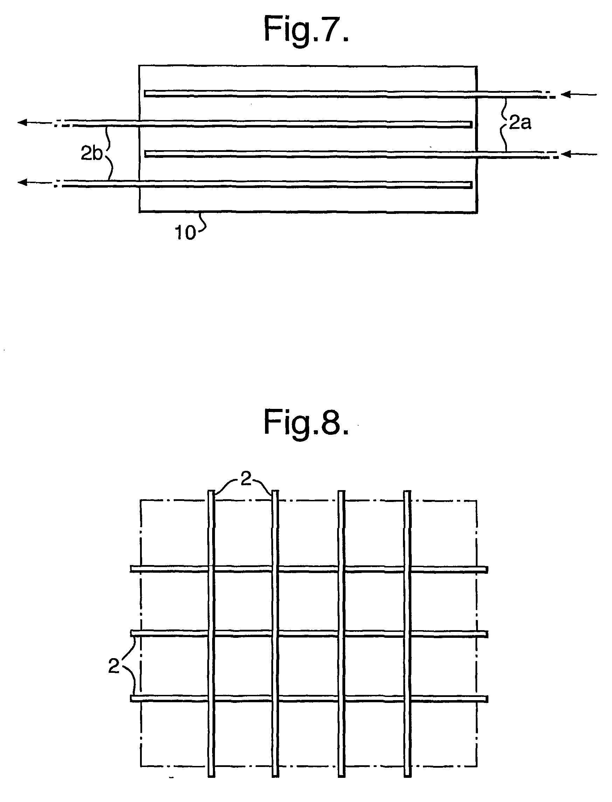

Figure 7 illustrates an alternative arrangement in which the membrane capillaries 2 are blocked at one end and open at the other end. With this arrangement, nutrient fluid may be provided in the open ends of some of the membrane capillaries 2a so as to feed the cells of the tissue. On the other hand, fluid may be extracted from the tissue and scaffold into others of the membrane capillaries 2b and removed from the structure.

It should be appreciated, of course, that the arrangements of Figures 6 and 7 can be combined using open ended and closed ended membrane capillaries 2 according to the particular needs of a particular tissue growth.

With regard to the arrangements of Figures 6 and 7, it should also be noted that the membrane capillaries 2 need not only be fed with nutrient fluid from one side and removed of metabolic waste from the other side. It is possible to locate the inlets and outlets at the same side or, indeed, at any appropriate position according to the particular needs of the tissue being grown. Indeed, capillaries may be arranged in any appropriate pattern, for instance, a grid as illustrated in Figure 8.

Figures 6, 7 and 8 illustrate particular arrangements whereby the membrane capillaries 2 are formed in the scaffold in predetermined organised patterns. In particular, the membrane capillaries are arranged parallel to one another with an even spacing. Clearly, this has some advantages in that an even distribution of nutrient and metabolic waste flow can be achieved.

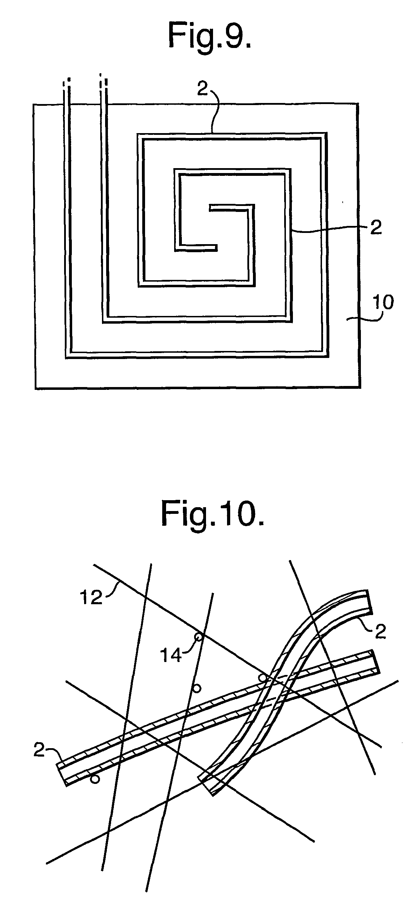

It should be appreciated that the membrane capillaries 2 need not only follow straight paths. In particular, they may follow any desired circuitous path through the scaffold and tissue. This is illustrated schematically in Figure 9.

Arrangements such as this have the advantage that inlets and outlets to membrane capillaries 2 may be grouped together and that fewer membrane capillaries 2 need be provided to supply nutrients and remove metabolic waste from a given volume of tissue. However, particular circuitous paths may be provided according to the particular needs of a particular type of tissue growth. Open ended and closed ended membrane capillaries 2 may be used and combined as described with reference to Figures 6, 7 and 8, indeed, straight and curved/shaped membrane capillaries 2 may be intermixed according to requirements.

It should also be noted that, provided cells are close enough to membrane capillaries 2 to receive nutrients and dispose of metabolic waste, the pattern of the membrane capillaries 2 may be unimportant. Hence, by providing a sufficient number and/or length of membrane capillaries 2 in a given volume of scaffold and tissue, it is possible to determine that all of the cells of the tissue will be close enough to appropriate membrane capillaries 2 to live and grow.

Hence, membrane capillaries 2 may be embedded, for instance by moulding, into the scaffold 10 in a random arrangement. In this case, it is still possible for the membrane capillaries to be open ended, closed ended or a mixture of the two. Similarly, of course, random and predetermined patterns of membrane capillaries 2 may also be mixed.

It is also possible to use capillaries which are blocked at both ends. This is illustrated in Figure 10.

The fluid inside the capillaries provides a diffusion path of reduced resistance compared to the growing tissue structure. Hence, the capillaries improve transfer of waste and nutrients through the structure. They also reserve space for the in-growth of blood vessels.

These capillaries which are blocked at both ends might preferably be used in conjunction with the capillaries discussed above and can have straight or circuitous paths in an organised or random pattern. However, it would also be possible to use only blocked capillaries. In this case, nutrients and waste will be diffused between the surface of the structure and portions of the capillaries in the region of the surface.

One way to make scaffold is to use polymer mesh, which consists of tangled polymer fibres. For example, the scaffold of artificial cartilage could use PLA fibre mesh as scaffold.

The hollow membrane capillaries can be used to supplement the scaffold materials. The capillaries (say around 0.1 - 0.3mm diameter) can be arranged or randomly packed. During the processing of the scaffold, the end of the capillaries may be blocked. Nutrients can diffuse into the membrane capillary and out depending on concentration gradients. Nutrient can flow inside the capillary if one or both ends are open and there exists a pressure gradient, or just diffuse through the liquid inside the capillary if both ends are blocked. The diffusion rate inside the capillaries would be much faster than through the forming tissue (by a factor of 2 at least, as there are no cells and ECM inside the capillary). The mechanism will help to maintain uniform distribution of nutrients and removal of waste within the 3-d tissue.

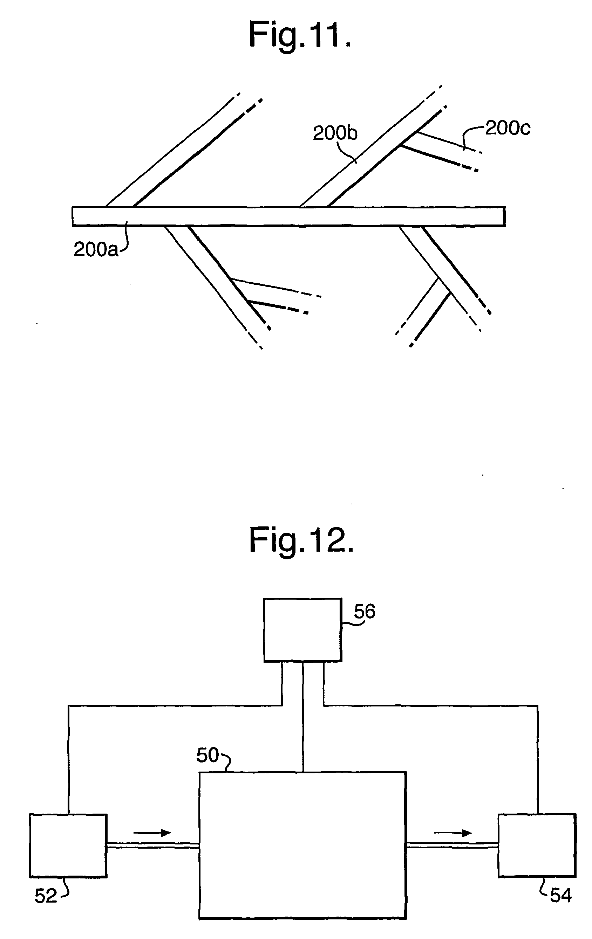

Figure 11 illustrates a further variation to the arrangements of Figures 6 to 10.

The capillaries may be interconnected to form a network. As illustrated, this takes the form of a tree structure of interconnected capillaries of different diameters. In a preferred embodiment, a main large diameter capillary 200a is connected to further smaller diameter capillaries 200b, themselves connected to subsidiary capillaries 200c.

As before, this arrangement can be mixed with those discussed above and take predetermined, random straight, circuitous, open ended or closed ended forms.

The minimum distance between membrane capillaries 2 to support life of the cells in the tissue will obviously vary according to the nature of the tissue. However, in normal cases for organic tissue such as animal and human tissue, the membrane capillaries 2 should be no further than 5mm apart and preferably approximately 0.5-1mm apart. Preferably, the membrane capillaries are positioned with no less than 0.5mm between adjacent respective walls.

The diffusion distance will depend on the scaffold and cell density. If just one set of capillaries is to provide nutrients and remove waste, the distance will be approximately 1-3 mm. If different capillaries are used for nutrient and waste, the distance should be approximately 1-5mm. Two capillaries supplying nutrient should be (roughly) 2 mm (or more as the third capillary also takes space) apart with a waste removal capillary between them. These numbers are just indicative. They are the distance between the two outside surfaces of the capillaries, as the capillary diameter may well be in the region of mm or more as described earlier.

The choice of capillary size also depends on the nature of the tissue being grown and the shape and size of the tissue being grown. Larger diameter capillaries provide greater flow of fluid and greater surface area per capillary through which the fluid may be dispersed or absorbed. On the other hand, larger diameter capillaries obviously take up more space in the tissue and, hence, the number of capillaries is limited.

For most applications, therefore, the membrane capillaries should be between 10µm and 2mm in diameter, preferably 0.1mm to 1mm.

As mentioned above, there is a possibility of introducing endothelial cells into or onto the capillaries 2.

It will be appreciated that, when a tissue structure has been grown and the membrane capillaries 2 degrade, the tissue structure is left with open channels. These channels with themselves convey nutrient and waste for some time. However, as the tissue continues to grow, the channels will become smaller until insufficient transfer of nutrients and waste occurs.

If the tissue structure is implanted into the appropriate animal or human at an early stage, it is possible for the tissue sample to develop blood vessels generated by the host body. However, as an alternative to this, it is possible to introduce endothelial cells into or onto the membrane capillaries. These cells could develop into blood vessels as the membrane capillaries degrade.

Figure 12 illustrates an overall system for growing the tissue. This includes a housing 50 for the structure discussed above whereby temperature and other environmental conditions can also be controlled, a nutrient supply device 52 and a waste disposal device 54. A controller 56 then controls the supply device 52 to supply nutrient fluid to the scaffold and tissue structure in the housing 50, controls the environmental conditions of the housing 50 and controls the waste disposal device 54 to extract the metabolic waste.

### Experimental Results

### Methods

### Cell Culture

Rat bone marrow fibroblastic cells were harvested using from femoral bone marrow of 3-month old female LOU/CN rats and pelleted by centrifugation at 1000rpm for 5 minutes. The cell pellet was resuspended in 10ml α-MEM (minimum essential medium) and passed through a cell strainer. Samples of cell suspension were diluted with 2% acetic acid in PBS (phosphate buffered saline) and the number and viability of nucleated cells were determined. Cells were plated at 1x10⁶ to 5x10⁶ in 25cm² flasks and cultured in 20mM buffered (-MEM supplemented with 10% (v/v) FBS, 1% antibiotics and 1% Fungizone.

After reaching a subconfluent state, cells were recovered by trypsin-EDTA (0.05% w/v) and cultured with Cytodex 1 microcarriers the day before inoculation.

### Microcarrier Bead Culture

As the rat bone marrow fibroblastic cells are anchorage-dependent cells, microcarrier beads Cytodex 1 were used as substrate for the cells to attach.

Cytodex 1 microcarriers are based on a cross-linked dextran matrix which is substituted with positively charged N, N-diethylaminoethyl (DEAE) groups. Cell cultures on Cytodex 1 were done following the protocol provided by the manufacturer.

### Preparation of Collagen Gel with Cells

* Trypsinise the requisite number of flasks of cells, spin down (i.e. centrifuge) and do cell counting. Calculate the number of cells that will be required and the necessary dilution.
* While the cells are in the centrifuge, put the ice in the hood and place the containers with the following solutions on the ice: 10xα-MEM, collagen, and 0.4M sodium hydroxide.
* Resuspend the cells and place the tube on ice.
* To prepare the collagen polymerising solution, dispense the following constituents in the order indicated. Mix by swirling gently after addition of each component.
   2ml distilled water
   1ml 10xα-MEM
   5ml collagen
* Add 0.4M NaOH dropwise using a Pasteur pipette until the solution goes orange on addition and swirling.
* Add 2ml of the cell suspension and swirl gently to mix thoroughly.

### Hollow Fibre Bioreactor

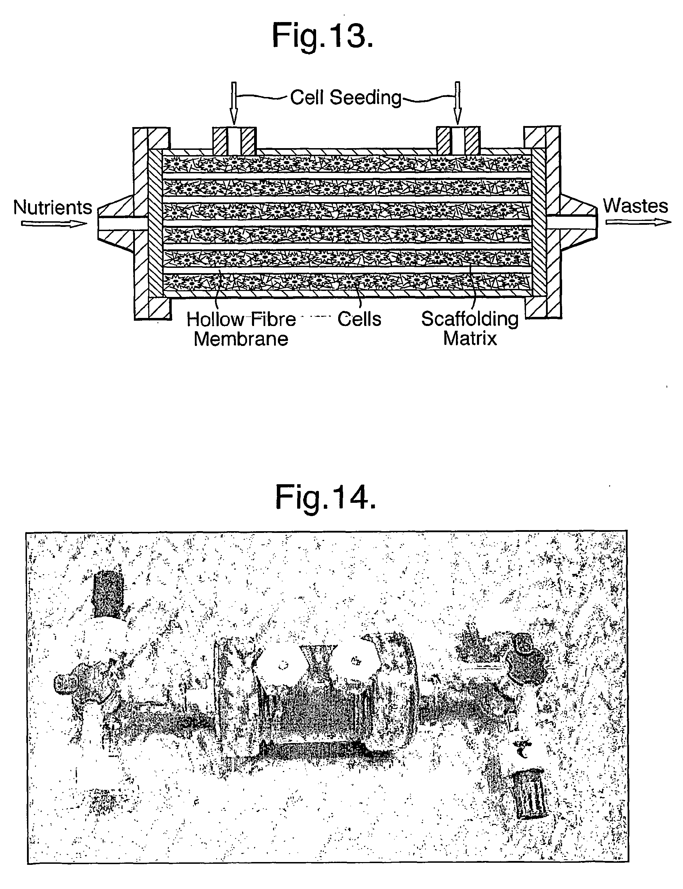

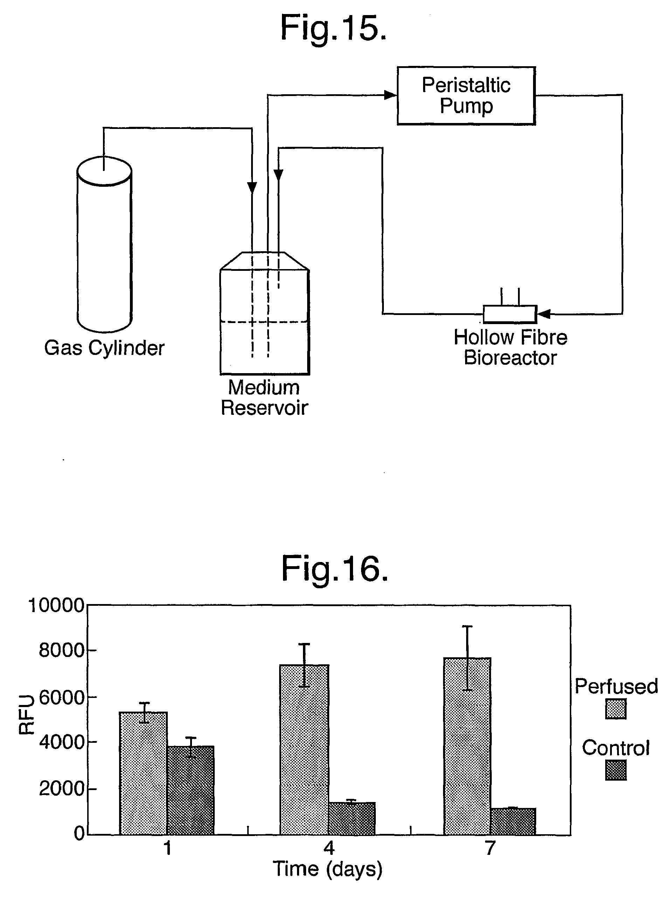

Figure 13 is a schematic representation of the hollow fibre bioreactor and Figure 14 illustrates the actual arrangement used for these experiments.

Two groups of membrane capillaries are embedded into the scaffolds of the tissue. The capillaries or hollow fibre membranes are of a diameter of 0.3mm to 1mm. The walls of these capillaries are porous and semi permeable. The first group of capillaries provides the function of arteries. The culture medium which is also oxygen rich flows inside this group of capillaries. The nutrients and oxygen flowing inside these fibres come out through the membrane pores and are distributed almost evenly within the tissue.

The nutrients flowing out of the first group of capillaries get into the cells outside and the cells consume nutrients and produce metabolites which need to be removed.

The second group of capillaries, which act as venules, are also embedded into the tissue and entangled with the first group of capillaries. The spent media and metabolic waste permeate into this second group of capillaries and flow out of the tissue. Therefore a nutrient circulation system similar to the capillary system in the native tissue is created.

The membrane pore size is not large enough for the cells to go through the membrane wall. Between the two sets of capillaries the scaffold materials, such as natural biopolymers or synthetic polymers, can be used to provide the scaffold for cell attachment. Some cells may attach to the membrane as well.

In the initial stage of cell and tissue culture the membrane pores will stop the cells going through the membrane. The membranes could be made of biodegradable polymers and could degrade as time progresses. Therefore the nominal pore size of these membranes could increase with time. This serves two functions:
1 As the culture progresses the cell density inside the tissue will increase greatly and hence a high transport of nutrients to the tissue is required. A larger pore size would help to meet this increased demand for nutrients.
2 In the later stages of the culture, endothelial cells can be introduced into the capillary systems both in the first group, that is the artery group, or the second group, the vein group. This is because the flow can easily be reversed with the second group to provide nutrients and the first group to collect the metabolic waste. The introduced endothelial cells could attach at the surface of these capillaries and form eventually capillary blood vessels. This particularly true when the membranes are originally introduced are degraded. It is expected that bulky tissue with an embedded capillary system may be produced by such procedures.

### Fabrication of Hollow Fibre Bioreactor

1 The cartridge (dimension 13mmIDx22mmODx40mmL) and side ports were processed in the workshop. The cartridges and screw caps on both ends were made of polycarbonate. The ports for medium (302) were clinical male luer connectors and the side ports for inoculation of cells (303) were clinical female luer connectors, which were glued onto the body of the cartridge.
2 Cellulose acetate hollow fibre membranes were cut out of normal haemodialyzers and fixed by silicon rubber at the both ends of the cartridge.

### Perfusion System

The perfusion system consisted of four major components: a hollow fibre bioreactor, a pump station, culture medium reservoir and gas cylinder (Fig. 15). Cells were inoculated into the ECS of the hollow fibre bioreactor. Culture medium was withdrawn from the medium reservoir by the peristaltic pump, entered the intracapillary space and then returned to the reservoir. Routinely a medium flow rate of 14ml/h was used.

### Control

A hollow fibre bioreactor with no medium flow served as control. The medium was changed on a daily basis in the control system.

### Maintenance of Bioreactors

150 ml culture medium was used for each bioreactor and changed on day 4. For the control system, medium was changed daily.

The pH value, partial pressure of CO₂ and O₂ and concentration of HCO₃-, Ca²+, K+, Na+, and Cl- of the culture medium were measured daily on a blood-gas analyzer. Lactate production of the cells in the bioreactors was measured by lactate reagent Sigma.

### Cell proliferation

Cell proliferation was measured using Alamar Blue assay. The Alamar Blue dye was reduced from a non-fluorescent form to a fluorescent product due to the metabolic activity of the cultured cells.

On day 1, 4 and 7, the medium circulation was stopped. After removing the medium remained in the bioreactor, fresh medium with 5% Alamar Blue was injected into the bioreactor. After 2hrs in a 37°C incubator, 200µl medium from each bioreactor was placed into 96-well plates. The relative fluorescent unit was measured by a fluorescent microplate reader at wavelengths of 530nm (excitation) and 590nm (emission). Controls containing only media and Alamar Blue reagent that had also been incubated for 2hrs were also measured.

### Cell viability

Cell viability was determined by a live/dead viability assay kit. After harvesting the bioreactor, the samples were rinsed in PBS and placed in a 24-well plate. 0.5ml working solution (2µM calcein and 4µM EthD-1 in PBS) were added to each well and incubated at 37°C for 45mins. Following incubation, several drops of PBS were added to a clean microscopic slide. Samples were transferred from 24-well plate to the microscopic slides and viewed under the fluorescence microscope (excitation/emission: calcein 494/517nm; EthD-1 528/617nm). Pictures of each sample were taken.

### H-E staining

After harvesting bioreactors, the samples were rinsed in PBS and fixed in 10% formaldehyde for 15mins. The samples were embedded in 2% agarose and then in paraffin and 4µm sections obtained.

Dewax sections in xylene for 15mins and rinse in 100%, 70% and 40% alcohol in turn. Rinse in running water for 5mins. Stain with Mayers haematoxylin for 20mins and wash and blue in running water for 5mins. Stain with eosin for 5mins and wash in running water for 1min. Dehydrate in 40%, 70% and 100% alcohol and clear in xylene. Mount in DPX mountant.

### SEM

The samples were fixed for SEM by using 3% glutaraldehyde and 1% osmic acid and dehydrated by using acetone of different concentrations (from 20% to 100%). Samples were subject to critical point drying using isoamyl acetate and then coated with gold. The samples were then observed by scanning electron microscope at 15kV acceleration voltage and pictures were taken.

### Results

### Cell proliferation

The Alamar blue assay for cell proliferation is shown in Fig 16. There is no difference between the control and the perfused at day1. The RFU of Alamar blue in the control group decreased at day 4 and day 7 compared with about 5 fold increases in the perfused group which is statistically significant (p < 0.05).

### Medium lactate concentration

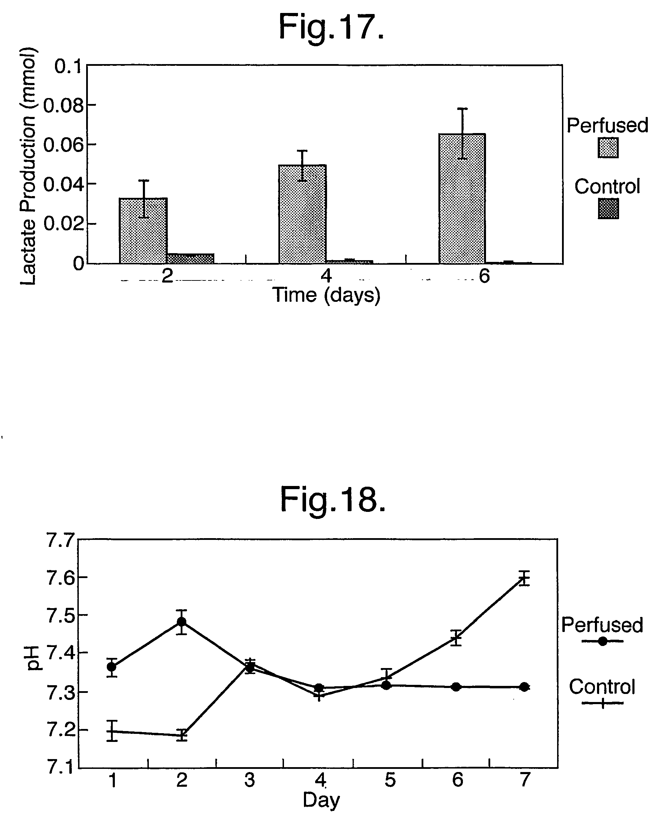

The results of lactate production in the culture medium in two groups are shown in Fig 17. The lactate produced by cells in control group is stable in all time points (between 0.00097 (0.00058 and 0.0044 (0.0004 mmol). However, in perfused bioreactor, the lactate in the culture medium is higher than the control group (between 0.032 (0.0091 and 0.065 (0.0129) significantly at day 4 and day 6 (p < 0.05).

### Medium gas and electrolytes analysis

The pH values of the medium in two groups are shown in Fig 18. The pH values in the perfused group is stable between 7.3 and 7.4 but had a slight increase to 7.48 (0.053. However, compared with the perfused group, the pH value in the control group increased at day 6 and day 7 to 7.44 (0.008 and 7.6 (0.03 (p < 0.05 and p < 0.01) respectively.

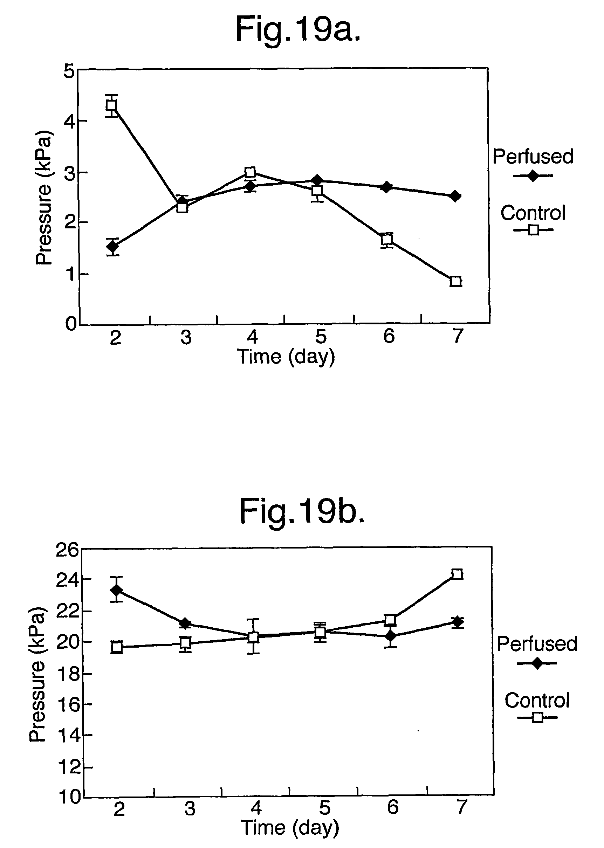

The partial pressures of 02 and CO2 in the two groups are shown in Fig 19. The results in the perfused group is relatively stable, but the control group showed a trend of slightly increasing in partial pressure of 02 and decreasing in partial pressure of CO2 between day 1 and day 7.

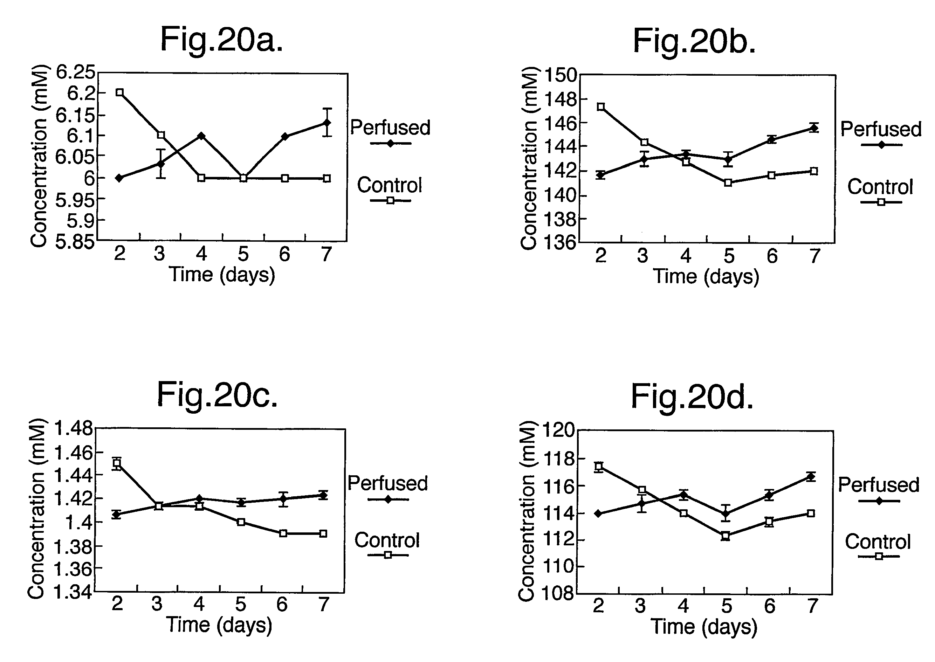

The K+, Na+, Ca++, Cl- concentrations in the medium of the two groups are variable but all are within physiological ranges (Fig 20).

### Cell viability

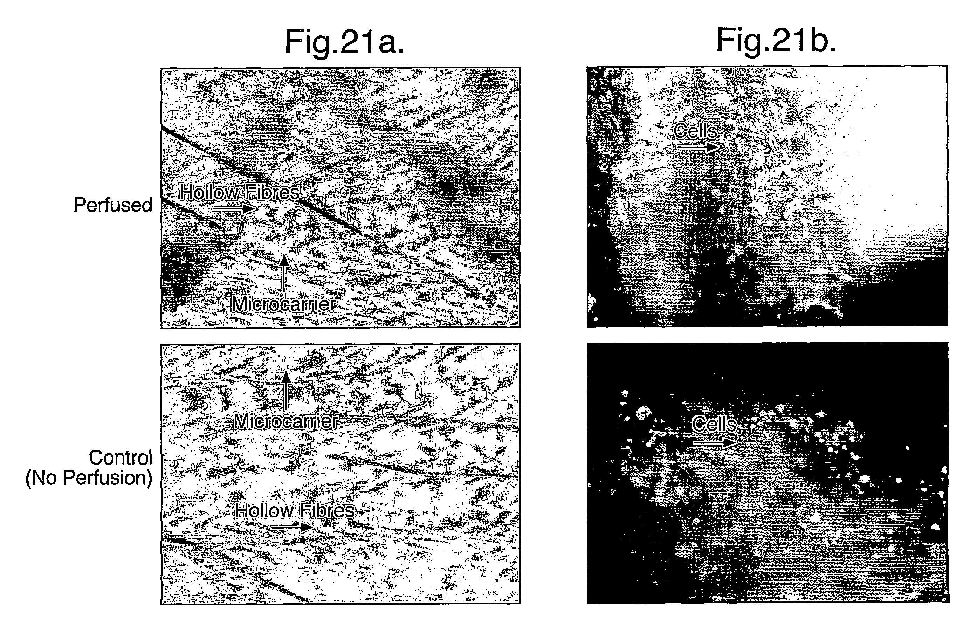

The live/dead cell staining is shown in Fig 21. The hollow fibres, collagen gel and cell carrier by light microscopy are illustrated in Fig 21A. After live/dead staining, the living cells are green and dead cells are red by fluorescence microscopy. In the perfused group, cells were evenly distributed over collagen gel matrix and microcarriers at high density. There were tissue-like structures formed. There were much more living cells in the perfused group than in the control. In the control group, cell density was lower and there were more dead cells than in perfused group (Fig. 21B).

### H&E staining

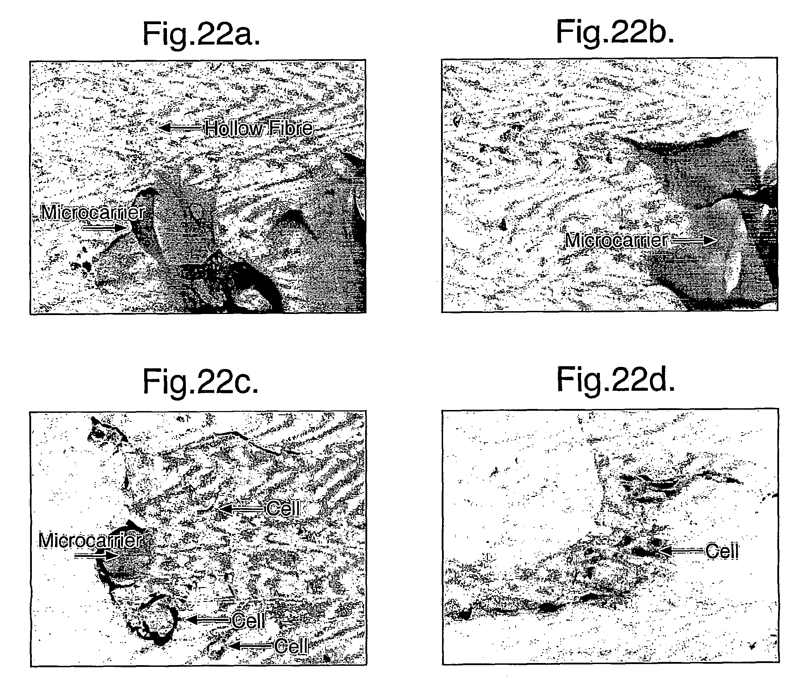

Fig. 22 shows H&E staining of harvested cells and tissue in hollow fibre bioreactors. Control group is shown in Fig 22 a and b. Cells were detached from the surface of cell carrier. Few cells survived in the collagen matrix. Perfused group is shown in Fig 22 c and d. Microcarriers were covered by cells. Cells evenly distributed in the collagen matrix and some three-dimensional tissue-like structures were formed.

### SEM observation

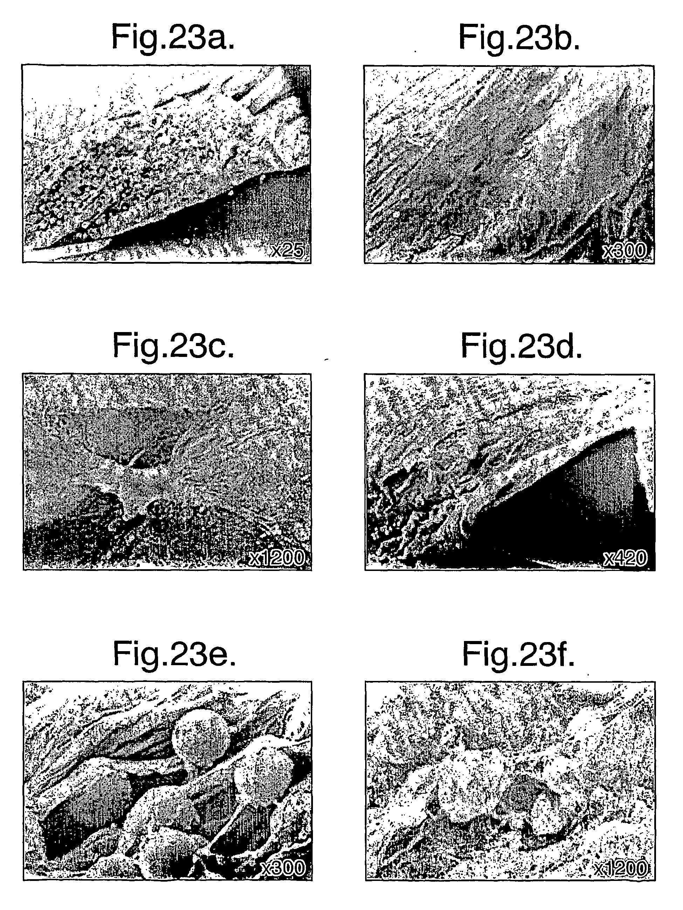

In both groups, the microcarriers were partially embedded into collagen-gel matrix that covered the hollow fibres and formed a compound structure (Fig 23 a). The surfaces of the hollow fibres were smooth with neither cells nor collagen matrix attached. Some cells grew on the microcarriers but they did not spread.

In the perfused group, cells formed a single layer throughout the outside surface of the collagen matrix, and multi-layer in some area. The cells were very active with elongated cell processes, which integrated into collagen matrix and formed cell junctions amongst cells (Fig 22 b). Some cells were forming new collagen matrix (Fig 23 c). There were no evidences showing cells grew between hollow fibres and collagen matrix (Fig 23d).

In the control group, the collagen matrix was not covered by cells. Some cells could be detected but they were in poor conditions, they did not spread like the cells in the perfused group. Dead cells and cell debris remained in the collagen matrix and microcarriers (Fig 23e). Compared with the organised fine collagen fibres that were orientated towards cells processes (Fig 22c), the collagen fibres in the control group were rough and disorganised without any relationship with cells and no signs of cell interference (Fig 23 f).

## Claims

1. A structure for growing living organic tissue including:
a scaffold (12) on which cells (14) of the tissue may be seeded and on which the tissue may be grown; and
a plurality of membrane capillaries (2), separate from the scaffold (12), each membrane capillary (2) including a respective membrane wall defining an internal passageway for conveying a nutrient fluid, wherein the membrane walls are permeable to nutrients and oxygen and the plurality of membrane capillaries are interspersed through the scaffold (12) along freely chosen paths such that the shape of the scaffold (12) may be freely chosen with the plurality of membrane capillaries (2) interspersed throughout the scaffold (12) so as to enable the tissue to be grown throughout the scaffold (12) wherein the membrane capillaries (2) are bio-degradable or bioresorbable.

2. A structure according to claim 1 wherein the scaffold (12) comprises a plurality of fibres of relatively small diameter compared to the plurality of membrane capillaries.

3. A structure according to claim 1 or 2 wherein the membrane capillaries (2) do not follow paths contained only within laminar sheet layers.

4. A structure according to claim 1, 2 or 3 wherein at least one of the membrane capillaries (2) follows a path which is neither in nor parallel with the laminar sheet layer in which the path of another of the membrane capillaries (2) lies.

5. A structure according to any preceding claim wherein at least one of the membrane capillaries (2) follows a convoluted path not contained within a laminar sheet layer.

6. A structure according to any preceding claim wherein the membrane capillaries (2) are omni-directional.

7. A structure according to any preceding claim wherein the scaffold (12) has an extent in three mutually orthogonal directions.

8. A structure according to any preceding claim wherein at least some of the membrane capillaries (2) are open at opposite ends so as each to provide a passage for flow of nutrient fluid through the tissue such that nutrient fluid provides nutrients to the tissue.

9. A structure according to any preceding claim wherein at least some of the membrane capillaries (2) are blocked at one end such that nutrient fluid may be supplied to the open end so as to provide nutrients to the tissue.

10. A structure according to any preceding claim wherein at least some of the membrane capillaries (2) are blocked at both ends.

11. A structure according to any preceding claim wherein at least some of the plurality of membrane capillaries (2) have membranes walls permeable to metabolic waste and are suitable for conveying fluid containing metabolic waste away from the tissue.

12. A structure according to any preceding claim further comprising an additional plurality of membrane capillaries (2) separate from the scaffold (12) and having membrane walls permeable to metabolic waste and which are interspersed through the scaffold (12) along freely chosen paths so as to convey metabolic waste fluid away from the tissue.

13. A structure according to claim 12 wherein at least some of the additional plurality of membrane capillaries (2) are blocked at one of opposite ends such that metabolic waste fluid may be drawn away from the tissue out of the open ends.

14. A structure according to claim 12 or 13 further including a waste extractor for drawing metabolic waste fluid from the additional plurality of membrane capillaries.

15. A structure according to any preceding claim wherein the membrane capillaries (2) are constructed from one of poly-lactic acid, poly-glycolic acid, poly-lactic-co-glycolic acid and poly caprolactone.

16. A structure according to any preceding claim wherein the membrane capillaries (2) are constructed from a material having pores which, as the membrane capillaries (2) degrade, increase in size so as to provide an increased supply of nutrient fluid to the tissue.

17. A structure according to any preceding claim wherein the membrane capillaries (2) are between 10µm and 2 mm diameter.

18. A structure according to claim 17 wherein the membrane capillaries (2) are between 0.1mm and 1mm diameter.

19. A structure according to any preceding claim wherein the membrane capillaries (2) are spaced no closer than 0.5 mm apart.

20. A structure according to claim 19 wherein the membrane capillaries (2) are approximately 1 mm apart.

21. A structure according to any preceding claim wherein at least some of the membrane capillaries (2) extend through the scaffold parallel to one another.

22. A structure according to any preceding claim wherein at least some of the membrane capillaries (2) follow respective circuitous paths through the scaffold.

23. A structure according to any preceding claim further including a nutrient fluid supply for providing nutrient fluid to the plurality of membrane capillaries (2).

24. A tissue structure including:
living organic tissue according to the structure of claims 1 to 23; and
and a plurality of artificial membrane capillaries (2) each membrane capillary (2) including a respective membrane wall defining an internal passageway for conveying a nutrient fluid, wherein the membrane walls are permeable to nutrients and oxygen and the plurality of membrane capillaries are interspersed through the tissue along freely chosen paths such that the shape of the tissue may be freely chosen with the plurality of membrane capillaries (2) interspersed throughout the tissue so as to support life of the tissue wherein the membrane capillaries (2) are bio-degradable or bioresorbable.

25. A method of growing living organic tissue including:
freely defining a volume of desired shape and size;
providing a plurality of membrane capillaries (2) along freely choosable paths interspersed through the volume, each membrane capillary (2) including a respective membrane wall which is permeable to nutrients and oxygen and which defines an internal passageway for conveying a nutrient fluid wherein the membrane capillaries (2) are bio-degradable or bioresorbable;
forming a scaffold (12) in the volume around the plurality of membrane capillaries (2), the scaffold (12) being suitable for seeding of tissue cells and tissue growth;
seeding cells of the tissue in the scaffold (12); and
providing a flow of nutrient fluid through the plurality of membrane capillaries (2).

26. A method according to claim 25 further including:
forming the scaffold (12) with a plurality of fibres of small diameter compared to the membrane capillaries (2).

27. A method according to claim 25 or 26 wherein the membrane capillaries (2) are not constrained to follow paths within laminar sheet layers.

28. A method according to claim 25, 26 or 27 further including:
providing at least one of the membrane capillaries (2) along a path which is neither in nor parallel with the laminar sheet layer in which the path of another of the membrane capillaries (2) lies.

29. A method according to any one of claims 25 to 28 further including:
providing at least one of the membrane capillaries (2) along a convoluted path not within a laminar sheet layer.

30. A method according to any one of claims 25 to 29 including:
providing the plurality of membrane capillaries (2) along omni-directional paths.

31. A method according to any one of claims 25 to 30 wherein said volume is defined by choosing a desired shape and size for a mould.

32. A method according to any one of claims 25 to 30 further including:
providing the plurality of membrane capillaries (2) in said mould.

33. A method according to any one of claims 25 to 32 further including:
introducing scaffold material into said mould around said plurality of membrane capillaries (2).

34. A method according to claim 33 further including mixing cells with the scaffold material before introducing the scaffold material into the mould.

35. A method according to any one of claims 25 to 34 further including:
introducing endothelial cells into the membrane capillaries (2) and stimulating growth of blood vessels in place of the membrane capillaries (2).

## Patentansprüche

1. Struktur zum Züchten von lebendem organischem Gewebe, die Folgendes beinhaltet:
ein Gerüst (12), auf welchem Zellen (14) des Gewebes eingeimpft werden können und auf welchem das Gewebe gezüchtet werden kann; und
eine Vielzahl an von dem Gerüst (12) getrennten Membrankapillaren (2), wobei jede Membrankapillare (2) eine jeweilige Membranwand beinhaltet, die einen inneren Durchgang zum Transport einer Nährflüssigkeit bzw. eines Nährstofffluids definiert, wobei die Membranwände für Nährstoffe und Sauerstoff durchlässig sind und das Gerüst (12) mit der Vielzahl an Membrankapillaren entlang frei gewählter Bahnen durchsetzt ist, sodass die Gestalt des Gerüsts (12) frei gewählt sein kann, wobei das Gerüst (12) mit der Vielzahl an Membrankapillaren (2) durchsetzt ist, um so das Gewebe in die Lage zu versetzen, überall im Gerüst (12) gezüchtet zu werden, wobei die Membrankapillaren (2) biologisch abbaubar oder biologisch resorbierbar sind.

2. Struktur nach Anspruch 1, wobei das Gerüst (12) eine Vielzahl an Fasern von relativ geringen kleinen Durchmesser im Vergleich mit der Vielzahl an Membrankapillaren umfasst.

3. Struktur nach Anspruch 1 oder 2, wobei die Membrankapillaren (2) nicht nur innerhalb laminarer Blattlagen enthaltenen Bahnen folgen.

4. Struktur nach Anspruch 1, 2 oder 3, wobei mindestens eine der Membrankapillaren (2) einer Bahn folgt, die sich weder in der laminaren Blattlage befindet noch parallel zu dieser verläuft, in welcher die Bahn einer weiteren der Membrankapillaren (2) liegt.

5. Struktur nach mindestens einem der vorhergehenden Ansprüche, wobei mindestens eine der Membrankapillaren (2) einer nicht innerhalb einer laminaren Blattlage enthaltenen gewundenen Bahn folgt.

6. Struktur nach mindestens einem der vorhergehenden Ansprüche, wobei die Membrankapillaren (2) omnidirektional sind.

7. Struktur nach mindestens einem der vorhergehenden Ansprüche, wobei das Gerüst (12) sich in drei jeweils zueinander orthogonalen Richtungen erstreckt.

8. Struktur nach mindestens einem der vorhergehenden Ansprüche, wobei wenigstens einige der Membrankapillaren (2) an gegenüberliegenden Enden offen sind, sodass jede davon einen Durchgang für einen Nährflüssigkeitsstrom durch das Gewebe hindurch vorsieht, sodass Nährflüssigkeit das Gewebe mit Nährstoffen versorgt.

9. Struktur nach mindestens einem der vorhergehenden Ansprüche, wobei wenigstens einige der Membrankapillaren (2) an einem Ende blockiert sind, sodass Nährflüssigkeit dem offenen Ende zugeführt werden kann, um so das Gewebe mit Nährstoffen zu versorgen.

10. Struktur nach mindestens einem der vorhergehenden Ansprüche, wobei wenigstens einige der Membrankapillaren (2) an beiden Enden blockiert sind.

11. Struktur nach mindestens einem der vorhergehenden Ansprüche, wobei wenigstens einige der Vielzahl an Membrankapillaren (2) Membranwände besitzen, die gegenüber metabolischen Abfallprodukten durchlässig sind und für das Abtransportieren von metabolische Abfallprodukte enthaltender Flüssigkeit aus dem Gewebe geeignet sind.

12. Struktur nach mindestens einem der vorhergehenden Ansprüche, weiterhin umfassend eine zusätzliche Vielzahl an von dem Gerüst (12) getrennten Membrankapillaren (2) und mit Membranwänden, die gegenüber metabolischen Abfallprodukten durchlässig sind, und mit denen das Gerüst (12) entlang frei gewählter Bahnen durchsetzt ist, um so metabolische Abfallflüssigkeit aus dem Gewebe abzutransportieren.

13. Struktur nach Anspruch 12, wobei wenigstens einige der zusätzlichen Vielzahl an Membrankapillaren (2) an einem von gegenüberliegenden Enden blockiert sind, sodass metabolische Abfallflüssigkeit aus dem Gewebe über die offenen Enden abgeführt werden kann.

14. Struktur nach Anspruch 12 oder 13, weiterhin einschließend einen Abfallprodukt-Extraktor zum Abführen von metabolischer Abfallflüssigkeit aus der zusätzlichen Vielzahl an Membrankapillaren.

15. Struktur nach mindestens einem der vorhergehenden Ansprüche, wobei die Membrankapillaren (2) aus einem von Polymilchsäure, Polyglykolsäure, Polymilchsäure-co-glykolsäure und Polycaprolacton aufgebaut sind.

16. Struktur nach mindestens einem der vorhergehenden Ansprüche, wobei die Membrankapillaren (2) aus einem Material mit Poren aufgebaut sind, die mit dem fortschreitenden Abbau der Membrankapillaren (2) an Größe zunehmen, um so für eine erhöhte Zufuhr von Nährflüssigkeit in das Gewebe zu sorgen.

17. Struktur nach mindestens einem der vorhergehenden Ansprüche, wobei die Membrankapillaren (2) zwischen 10 µm und 2 mm Durchmesser haben.

18. Struktur nach Anspruch 17, wobei die Membrankapillaren (2) zwischen 0,1 mm und 1 mm Durchmesser haben.

19. Struktur nach mindestens einem der vorhergehenden Ansprüche, wobei der Abstand zwischen den Membrankapillaren (2) nicht weniger als 0,5 mm beträgt.

20. Struktur nach Anspruch 19, wobei der Abstand zwischen den Membrankapillaren (2) ungefähr 1 mm beträgt.

21. Struktur nach mindestens einem der vorhergehenden Ansprüche, wobei wenigstens einige der Membrankapillaren (2) durch das Gerüst hindurch parallel zueinander verlaufen.

22. Struktur nach mindestens einem der vorhergehenden Ansprüche, wobei wenigstens einige der Membrankapillaren (2) auf Umwegen über jeweilige Bahnen durch das Gerüst verlaufen.

23. Struktur nach mindestens einem der vorhergehenden Ansprüche, weiterhin einschließend eine Nährflüssigkeitszufuhr, um die Vielzahl an Membrankapillaren (2) mit Nährflüssigkeit zu versorgen.

24. Gewebestruktur, die Folgendes beinhaltet:
Lebendes organisches Gewebe gemäß der Struktur der Ansprüche 1 bis 23; und
eine Vielzahl an künstlichen Membrankapillaren (2), wobei jede Membrankapillare (2) eine jeweilige Membranwand beinhaltet, die einen inneren Durchgang zum Transport einer Nährflüssigkeit definiert, wobei die Membranwände für Nährstoffe und Sauerstoff durchlässig sind und das Gewebe mit der Vielzahl an Membrankapillaren entlang frei gewählter Bahnen durchsetzt ist, sodass die Gestalt des Gewebes frei gewählt sein kann, wobei das Gewebe mit der Vielzahl an Membrankapillaren (2) durchsetzt ist, um so das Leben des Gewebes zu unterstützen, wobei die Membrankapillaren (2) biologisch abbaubar oder biologisch resorbierbar sind.

25. Verfahren zum Züchten von lebendem organischem Gewebe, das Folgendes beinhaltet:
Freies Definieren eines Volumens mit der gewünschten Gestalt und Größe;
Vorsehen einer Vielzahl an Membrankapillaren (2) entlang frei wählbarer Bahnen, mit denen das Volumen durchsetzt ist, wobei jede Membrankapillare (2) eine jeweilige Membranwand einschließt, welche gegenüber Nährstoffen und Sauerstoff durchlässig ist, und welche einen inneren Durchgang für den Transport einer Nährflüssigkeit definiert, wobei die Membrankapillaren (2) biologisch abbaubar oder biologisch resorbierbar sind;
Bilden eines Gerüsts (12) in dem Volumen um die Vielzahl an Membrankapillaren (2) herum, wobei das Gerüst (12) für das Einimpfen von Gewebezellen und das Gewebewachstum geeignet ist;
Einimpfen von Zellen des Gewebes in das Gerüst (12); und
Vorsehen eines Nährflüssigkeitsstroms durch die Vielzahl an Membrankapillaren (2) hindurch.

26. Verfahren nach Anspruch 25, weiterhin einschließend:
Bilden des Gerüsts (12) mit einer Vielzahl an Fasern von kleinem Durchmesser im Vergleich mit den Membrankapillaren (2).

27. Verfahren nach Anspruch 25 oder 26, wobei die Membrankapillaren (2) nicht zwangsweise Bahnen innerhalb laminarer Blattlagen folgen.

28. Verfahren nach Anspruch 25, 26 oder 27, weiterhin einschließend:
Vorsehen von mindestens einer der Membrankapillaren (2) entlang einer Bahn, die sich weder in der laminaren Blattlage befindet noch parallel zu dieser verläuft, in welcher die Bahn einer weiteren der Membrankapillare (2) liegt.

29. Verfahren nach mindestens einem der vorhergehenden Ansprüche 25 bis 28, weiterhin einschließend:
Vorsehen mindestens einer der Membrankapillaren (2) entlang einer nicht innerhalb einer laminaren Blattlage liegenden gewundenen Bahn.

30. Verfahren nach mindestens einem der Ansprüche 25 bis 29, einschließend:
Vorsehen der Vielzahl an Membrankapillaren (2) entlang omnidirektionaler Bahnen.

31. Verfahren nach mindestens einem der Ansprüche 25 bis 30, wobei das Volumen durch Wählen einer gewünschten Gestalt und Größe für eine Form definiert ist.

32. Verfahren nach mindestens einem der Ansprüche 25 bis 30, weiterhin einschließend:
Vorsehen der Vielzahl an Membrankapillaren (2) in der besagten Form.

33. Verfahren nach mindestens einem der Ansprüche 25 bis 32, weiterhin einschließend:
Einführen von Gerüstmaterial in die Form um die Vielzahl an Membrankapillaren (2) herum.

34. Verfahren nach Anspruch 33, weiterhin einschließend das Mischen von Zellen mit dem Gerüstmaterial vor dem Einführen des Gerüstmaterials in die Form.

35. Verfahren nach mindestens einem der Ansprüche 25 bis 34, weiterhin einschließend:
Einführen von Endothelzellen in die Membrankapillaren (2) und Stimulieren des Wachstums von Blutgefäßen an Stelle der Membrankapillaren (2).

## Revendications

1. Structure pour cultiver un tissu organique vivant, comprenant :
un échafaudage (12) sur lequel des cellules (14) du tissu peuvent être ensemencées et sur lequel le tissu peut être cultivé ; et
une pluralité de capillaires (2) à membrane, séparés de l'échafaudage (12), chaque capillaire (2) à membrane comprenant une paroi de membrane respective définissant une voie de passage interne pour transporter un fluide nutritif, dans laquelle les parois de membrane sont perméables aux substances nutritives et à l'oxygène, et la pluralité de capillaires à membrane sont dispersés à travers l'échafaudage (12) le long de passages choisis librement, de sorte que la forme de l'échafaudage (12) peut être choisie librement avec une pluralité de capillaires (2) à membrane dispersés sur tout l'échafaudage (12) pour permettre au tissu d'être cultivé sur tout l'échafaudage (12), les capillaires (2) à membrane étant biodégradables ou biorésorbables.

2. Structure selon la revendication 1, dans laquelle l'échafaudage (12) comprend une pluralité de fibres de diamètre relativement petit par rapport à la pluralité de capillaires à membrane.

3. Structure selon la revendication 1 ou 2, dans laquelle les capillaires (2) à membrane ne suivent pas les passages contenus uniquement à l'intérieur de couches de feuille laminaires.

4. Structure selon la revendication 1, 2 ou 3, dans laquelle au moins l'un des capillaires (2) à membrane suit un passage qui n'est ni dans, ni parallèle à la couche de feuille laminaire, dans laquelle le passage d'un autre des capillaires (2) à membrane se trouve.

5. Structure selon l'une quelconque des revendications précédentes, dans laquelle au moins l'un des capillaires (2) à membrane suit un passage convoluté non contenu à l'intérieur de la couche de feuille laminaire.

6. Structure selon l'une quelconque des revendications précédentes, dans laquelle les capillaires (2) à membrane sont omnidirectionnels.

7. Structure selon l'une quelconque des revendications précédentes, dans laquelle l'échafaudage (12) a une étendue dans trois directions mutuellement orthogonales.

8. Structure selon l'une quelconque des revendications précédentes, dans laquelle au moins certains des capillaires (2) à membrane sont ouverts à des extrémités opposées, de façon à assurer chacun un passage pour l'écoulement du fluide nutritif à travers le tissu, de sorte que le fluide nutritif fournit des substances nutritives au tissu.

9. Structure selon l'une quelconque des revendications précédentes, dans laquelle au moins certains des capillaires (2) à membrane sont bloqués à une extrémité, de sorte que le fluide nutritif peut être alimenté vers l'extrémité ouverte, afin de fournir des substances nutritives au tissu.

10. Structure selon l'une quelconque des revendications précédentes, dans laquelle au moins certains des capillaires (2) à membrane sont bloqués aux deux extrémités.

11. Structure selon l'une quelconque des revendications précédentes, dans laquelle au moins certains de la pluralité de capillaires (2) à membrane ont des parois de membrane perméables aux déchets métaboliques, et sont appropriés pour transporter le fluide contenant les déchets métaboliques à distance du tissu.

12. Structure selon l'une quelconque des revendications précédentes, comprenant en outre une pluralité additionnelle de capillaires (2) à membrane séparés de l'échafaudage (12) et ayant des parois de membrane perméables aux déchets métaboliques et qui sont dispersés à travers l'échafaudage (12) le long de passages librement choisis, afin de transporter le fluide de déchets métaboliques à l'écart du tissu.

13. Structure selon la revendication 12, dans laquelle au moins certains de la pluralité additionnelle de capillaires (2) à membrane sont bloqués à l'une d'extrémités opposées, de sorte que le fluide de déchets métaboliques peut être aspiré à l'écart du tissu à travers les extrémités ouvertes.

14. Structure selon la revendication 12 ou 13, comprenant en outre un extracteur de déchets pour aspirer le fluide de déchets métaboliques à partir de la pluralité additionnelle de capillaires à membrane.

15. Structure selon l'une quelconque des revendications précédentes, dans laquelle les capillaires (2) à membrane sont construits à partir de l'un parmi l'acide polylactique, l'acide polyglycolique, l'acide polylactique coglycolique, et la polycaprolactone.

16. Structure selon l'une quelconque des revendications précédentes, dans laquelle les capillaires (2) à membrane sont construits à partir d'un matériau ayant des pores qui, lorsque les capillaires (2) à membrane se dégradent, augmentent en taille afin d'assurer une alimentation accrue de fluide nutritif au tissu.

17. Structure selon l'une quelconque des revendications précédentes, dans laquelle les capillaires (2) à membrane ont un diamètre compris entre 10 µm et 2 mm.

18. Structure selon la revendication 17, dans laquelle les capillaires (2) à membrane ont un diamètre compris entre 0,1 mm et 1 mm.

19. Structure selon l'une quelconque des revendications précédentes, dans laquelle les capillaires (2) à membrane sont espacés d'une distance non inférieure à 0,5 mm.

20. Structure selon la revendication 19, dans laquelle les capillaires (2) à membrane sont approximativement espacés de 1 mm.

21. Structure selon l'une quelconque des revendications précédentes, dans laquelle au moins certains des capillaires (2) à membrane s'étendent à travers l'échafaudage parallèlement les uns aux autres.

22. Structure selon l'une quelconque des revendications précédentes, dans laquelle au moins certains des capillaires (2) à membrane suivent des passages tortueux respectifs à travers l'échafaudage.

23. Structure selon l'une quelconque des revendications précédentes, comprenant en outre une alimentation de fluide nutritif pour délivrer le fluide nutritif à la pluralité de capillaires (2) à membrane.

24. Structure de tissu, comprenant :
un tissu organique vivant selon la structure des revendications 1 à 23 ;
et une pluralité de capillaires (2) artificiels à membrane, chaque capillaire (2) à membrane comprenant une paroi de membrane respective définissant une voie de passage interne pour transporter un fluide nutritif, dans laquelle les parois de membrane sont perméables aux substances nutritives et à l'oxygène, et la pluralité de capillaires à membrane sont dispersés à travers le tissu le long de passages librement choisis, de sorte que la forme du tissu peut être librement choisie avec la pluralité de capillaires (2) à membrane dispersés sur tout le tissu afin de maintenir la vie du tissu, dans lequel les capillaires (2) à membrane étant biodégradables ou biorésorbables.

25. Procédé de culture d'un tissu organique vivant, comprenant les étapes consistant à :
définir librement un volume d'une forme et d'une taille souhaitées ;
prévoir une pluralité de capillaires (2) à membrane le long de passages pouvant être librement choisis, dispersés à travers le volume, chaque capillaire (2) à membrane comprenant une paroi de membrane respective qui est perméable aux substances nutritives et à l'oxygène et qui définit une voie de passage interne pour transporter un fluide nutritif, les capillaires (2) à membrane étant biodégradables ou biorésorbables ;
former un échafaudage (12) dans le volume situé autour de la pluralité de capillaires (2) à membrane, l'échafaudage (12) étant approprié pour ensemencer des cellules tissulaires et cultiver le tissu ;
ensemencer des cellules du tissu dans l'échafaudage (12) ; et
réaliser un écoulement du fluide nutritif à travers la pluralité de capillaires (2) à membrane.

26. Procédé selon la revendication 25, comprenant en outre l'étape consistant à :
former l'échafaudage (12) avec une pluralité de fibres de petit diamètre comparé aux capillaires (2) à membrane.

27. Procédé selon la revendication 25 ou 26, dans lequel les capillaires (2) à membrane ne sont pas obligés de suivre les passages à l'intérieur de couches de feuille laminaires.

28. Procédé selon la revendication 25, 26 ou 27, comprenant en outre l'étape consistant à :
prévoir au moins l'un des capillaires (2) à membrane le long d'un passage qui n'est ni dans, ni parallèle à la couche de feuille laminaire, dans laquelle le passage d'un autre capillaire des capillaires (2) à membrane se trouve.

29. Procédé selon l'une quelconque des revendications 25 à 28, comprenant en outre l'étape consistant à :
prévoir au moins l'un des capillaires (2) à membrane le long d'un passage convoluté non situé à l'intérieur d'une couche de feuille laminaire.

30. Procédé selon l'une quelconque des revendications 25 à 29, comprenant l'étape consistant à :
prévoir la pluralité de capillaires (2) à membrane le long de passages omnidirectionnels.

31. Procédé selon l'une quelconque des revendications 25 à 30, dans lequel ledit volume est défini en choisissant une forme et une taille souhaitées pour un moule.

32. Procédé selon l'une quelconque des revendications 25 à 30, comprenant en outre l'étape consistant à :
prévoir la pluralité des capillaires (2) à membrane dans ledit moule.

33. Procédé selon l'une quelconque des revendications 25 à 32, comprenant en outre l'étape consistant à :
introduire le matériau d'échafaudage dans ledit moule autour de ladite pluralité de capillaires (2) à membrane.

34. Procédé selon la revendication 33, comprenant en outre l'étape consistant à mélanger des cellules avec le matériau d'échafaudage avant d'introduire le matériau d'échafaudage dans le moule.

35. Procédé selon l'une quelconque des revendications 25 à 34, comprenant en outre l'étape consistant à :
introduire des cellules endothéliales dans les capillaires (2) à membrane et stimuler le développement des vaisseaux sanguins à la place des capillaires (2) à membrane.
